# EUROPÄISCHE PATENTSCHRIFT

(11) **EP 2 812 317 B1**
(45) Veröffentlichungstag und Bekanntmachung des Hinweises auf die Patenterteilung: **03.02.2016**
(21) Anmeldenummer: 13703045.8
(22) Anmeldetag: 06.02.2013
(51) Int. Cl.: C07D 241/38, C07D 491/113

(54) **VERFAHREN ZUR STEREOSELEKTIVEN SYNTHESE VON 1,4-GESCHÜTZTEN 9-HYDROXY-5-OXO-1,4-DIAZA-SPIRO [5.5]UNDECANE**
METHOD FOR STEREOSELECTIVE SYNTHESIS OF 1,4-PROTECTED 9-HYDROXY-5-OXO-1,4-DIAZA-SPIRO [5.5]UNDECANES
PROCÉDÉ DE SYNTHÈSE STÉRÉOSÉLECTIVE DE 9-HYDROXY-5-OXO-1,4-DIAZA-SPIRO [5.5]UNDÉCANES QUI SONT 1,4-PROTÉGÉS

(30) Priorität: 09.02.2012 EP 12154688
(43) Veröffentlichungstag der Anmeldung: 17.12.2014
(73) Patentinhaber: Boehringer Ingelheim International GmbH, 55216 Ingelheim (DE)
(72) Erfinder: SCHNAUBELT, Juergen, 55216 Ingelheim am Rhein (DE); TANG, Wenjun, Ridgefield, Connecticut 06877 (US)
(74) Vertreter: Simon, Elke Anna Maria
(86) Internationale Anmeldenummer: PCT/EP2013/052287
(87) Internationale Veröffentlichungsnummer: WO 2013/117568

(56) Entgegenhaltungen:
- WO-A1-2009/098061
- WO-A1-2011/015526
- WO-A1-2012/104206

## Beschreibung

Gegenstand der Erfindung ist ein Verfahren zur stereoselektiven Herstellung einer Verbindung der Formel (5), gegebenenfalls in Form ihrer Tautomeren,

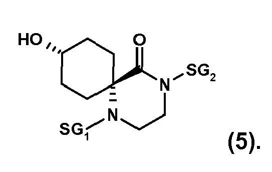

### HINTERGRUND DER ERFINDUNG

Verbindungen der Formel (5), gegebenenfalls in Form ihrer Tautomeren, sind wertvolle Intermediate in Verfahren zur stereoselektiven Herstellung der Verbindung der Formel (I), gegebenenfalls in Form ihrer Tautomeren, sowie gegebenenfalls ihrer pharmakologisch unbedenklichen Säureadditionssalze, 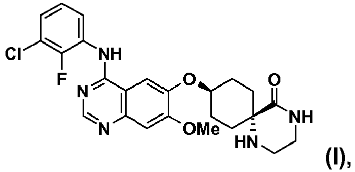 welche wertvolle pharmakologische Eigenschaften aufweist, insbesondere eine Hemmwirkung auf die durch Tyrosinkinasen vermittelte Signaltransduktion, und zur Behandlung von Krankheiten, insbesondere von Tumorerkrankungen, der benignen Prostatahyperplasie sowie von Erkrankungen der Lunge und der Atemwege geeignet ist.

Chinazolinderivate sind aus dem Stand der Technik als Wirkstoffe beispielsweise zur Behandlung von Tumorerkrankungen als auch von Erkrankungen der Lunge und der Atemwege bekannt. Verfahren zur Herstellung von Chinazolinderivaten werden in WO03082290 und WO07068552 beschrieben. WO2009098061 und WO2011015526 offenbaren Verfahren zur Herstellung der Verbindung (I).

Es ist die Aufgabe der vorliegenden Erfindung ein alternatives stereoselektives Syntheseverfahren zur Herstellung der Verbindung der Formel (I) bzw. ein vorteilhaftes Verfahren zur Herstellung wichtiger Intermediate dieser Synthese bereitzustellen.

### DETAILLIERTE BESCHREIBUNG DER ERFINDUNG

Die vorliegende Erfindung löst die vorstehend genannte Aufgabe über das im Folgenden beschriebene Syntheseverfahren.

Gegenstand der Erfindung ist ein Verfahren zur stereoselektiven Herstellung der Verbindung der Formel (5), gegebenenfalls in Form ihrer Tautomeren, 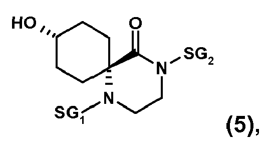 worin
**SG₁** eine Schutzgruppe ausgewählt aus der Gruppe bestehend aus Trifluoracetyl, Acetyl, Benzoyl und Pivaloyl,
**SG₂** eine Schutzgruppe ausgewählt aus der Gruppe bestehend aus tert.-Butoxycarbonyl, Ethoxycarbonyl und Methoxycarbonyl,
bedeutet,
dadurch gekennzeichnet, dass das Verfahren die Reaktionsschritte (C) und (D) umfasst, wobei
**(C)** die Umsetzung der Verbindung der Formel (3) 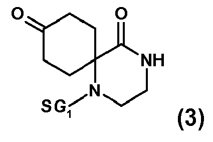 zu einer Verbindung der Formel **(4)** 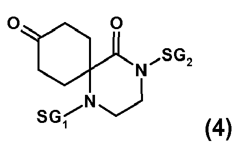 und
**(D)** die Reduktion der Verbindung der Formel **(4)** zu einer Verbindung der Formel **(5)**
   bedeutet,
   wobei die Verfahrensschritte **(C)** und **(D)** in der genannten Reihenfolge nacheinander erfolgen.

Bevorzugt ist ein Verfahren bei welchem den Reaktionsschritten **(C)** und **(D)** weitere Reaktionsschritte **(A)** und **(B)** vorausgehen,
wobei
**(A)** die Umsetzung der Verbindung der Formel **(1)** 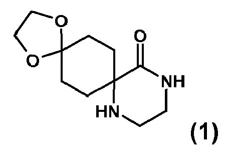 zu einer Verbindung der Formel **(2)** 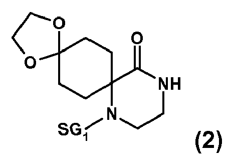 und
**(B)** die Umsetzung der Verbindung der Formel (2) zu einer Verbindung der Formel **(3)** 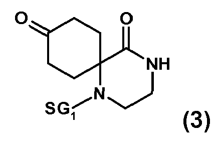 bedeutet,
   wobei die Verfahrensschritte **(A), (B), (C)** und **(D)** in der genannten Reihenfolge nacheinander erfolgen.

Weiterhin bevorzugt ist ein Verfahren zur stereoselektiven Herstellung der Verbindung der Formel (5), gegebenenfalls in Form ihrer Tautomeren, 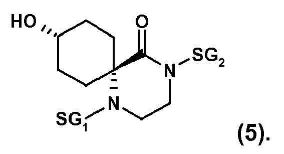 dadurch gekennzeichnet, dass
- **SG₁**: Trifluoracetyl, und
- **SG₂**: tert.-Butoxycarbonyl,
bedeutet.

Ein weiterer Gegenstand der Erfindung ist ein Intermediat der Formel **(4),** gegebenenfalls in Form seiner Tautomeren oder Salze. 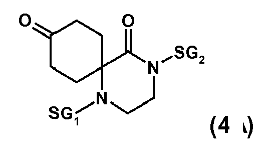 worin
- **SG₁**: eine Schutzgruppe ausgewählt aus der Gruppe bestehend aus Trifluoracetyl, Acetyl, Benzoyl und Pivaloyl, und
- **SG₂**: eine Schutzgruppe ausgewählt aus der Gruppe bestehend aus tert.- Butoxycarbonyl, Ethoxycarbonyl und Methoxycarbonyl,
bedeutet.

Bevorzugt ist ein Intermediat der Formel **(4A),** gegebenenfalls in Form seiner Tautomeren oder Salze.

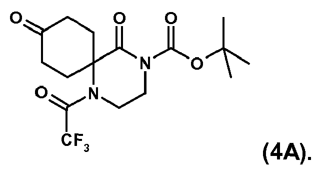

Ein weiterer Gegenstand der Erfindung ist die Verwendung der Intermediate der Formel **(4)**, vorzugsweise der Intermediate **(4A)**, gegebenenfalls in Form ihrer Tautomeren oder Salze, zur Herstellung einer Verbindung der Formel (I), gegebenenfalls in Form ihrer Tautomeren oder Salze.

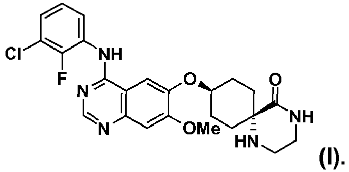

Die erfindungsgemäße Verwendung der Intermediate **(4)**, vorzugsweise der Intermediate **(4A)**, zur Herstellung einer Verbindung der Formel (I) kann über die Herstellung der Verbindung der Formel (5) und den darauffolgenden in WO2012104206 beschriebenen Syntheseschritten (G) bis (L) erfolgen.
Gegenüber der in WO2012104206 beschriebenen Synthese ergeben sich folgende Vorteile, insbesondere im Hinblick auf eine großtechnische Herstellung der Verbindung (I):
a) Die Gesamtausbeute der Verbindung **(5)** ausgehend von Verbindung **(1)** wird deutlich zwischen 10 bis 20 %, beispielsweise um 15 % auf 60% erhöht.
b) Es werden geringere Mengen Essigsäure zur Acetalspaltung eingesetzt und damit verbundene größerer Mengen saurer Abfälle vermieden.
c) Der Einsatz von Natriumborhydrid wird durch Verwendung eines kostengünstigeren heterogen katalytischen Hydrierverfahrens vermieden.
In den Verfahrensschritten **(A), (B), (C), (D)** können alternative Reagenzien, Katalysatoren und Lösungsmittel vorzugsweise ausgewählt aus der Gruppe bestehend aus den in Tabelle I.1 bis I.4 gelisteten Reagenzien, Katalysatoren und Lösungsmittel eingesetzt werden:

**Tabelle I.1 Alternative Lösungsmittel**

| **Verfahrensschritt** | **Lösungsmittel** | **Insbesondere bevorzugte Lösungsmittel** |
|---|---|---|
| **(A)** | Me-THF, THF, Toluol, DMF, NMP, Ethylacetat, Methylcyclohexan, Cyclohexan, CH₂Cl₂ und Dioxan | Me-THF und Methylcyclohexan |
| **(B)** | Wasser, Methanol, Ethanol, Isopropanol und Dioxan | Wasser und Methanol |
| **(C)** | Me-THF, THF, DMF, NMP, Aceton, Acetonitril und Dioxan | Acetonitril |
| **(D)** | Methanol, Ethanol, Isopropanol, Wasser und Ethylacetat | Ethanol |

**Tabelle I.2 Alternative Basen und Säuren**

| **Verfahrensschritt** | | **Basen bzw. Säuren** | **Insbesondere bevorzugte Basen bzw. Säuren** |
|---|---|---|---|
| **(A)** | Basen | N-Methylmorpholin, Triethylamin, N-Ethyldiisopropylamin (Hünig-Base), N-Methylpyrrolidin und N-Methylpiperidin | N-Methylmorpholin |
| **(B)** | Säuren | Essigsäure, Trifluoressigsäure, Salzsäure, Schwefelsäure, Methansulfonsäure und p-Toluolsulfonsäure | Essigsäure |

**Tabelle I.3 Alternative Reagenzien**

| **Verfahrensschritt** | **Reagenzien** | **Insbesondere bevorzugte Reagenzien** |
|---|---|---|
| **(A)** | Trifluoressigsäureanhydrid, Trifluoressigsäuremethylester, Trifluoracetylchlorid und Trifluoracetylimidazol | Trifluoressigsäureanhydrid, |
| **(C)** | Di-tert-butyldicarbonat | Di-tert-butyldicarbonat |

**Tabelle 1.4 Alternative Katalysatoren**

| **Verfahrensschritt** | **Katalysatoren** | **Insbesondere bevorzugte Katalysatoren** |
|---|---|---|
| **(C)** | Dimethylaminopyridin (DMAP) und Pyridin | DMAP |
| **(D)** | Raney-Nickel, Platin/Kohle, Platindioxid, Ruthenium/Kohle und Rhodium/Kohle | Raney-Nickel |

Die oben beschriebenen Verfahrensschritte **(A)** bis **(D)** werden vorzugsweise in folgenden Temperaturbereichen durchgeführt:
In Verfahrensschritt:
   **(A):** vorzugsweise 0 bis 70°C, besonders bevorzugt 10 bis 20°C;
   **(B):** vorzugsweise 20 bis70°C, besonders bevorzugt 60 bis 70°C;
   **(C):** vorzugsweise 0 bis 78°C, besonders bevorzugt 10.bis 30°C;
   **(D):** vorzugsweise 20 bis 80 °C, besonders bevorzugt 40. bis 70°C.

Schema 1 illustriert die erfindungsgemäße Synthese. Sämtliche Verbindungen sind in Form ihrer Basen dargestellt.

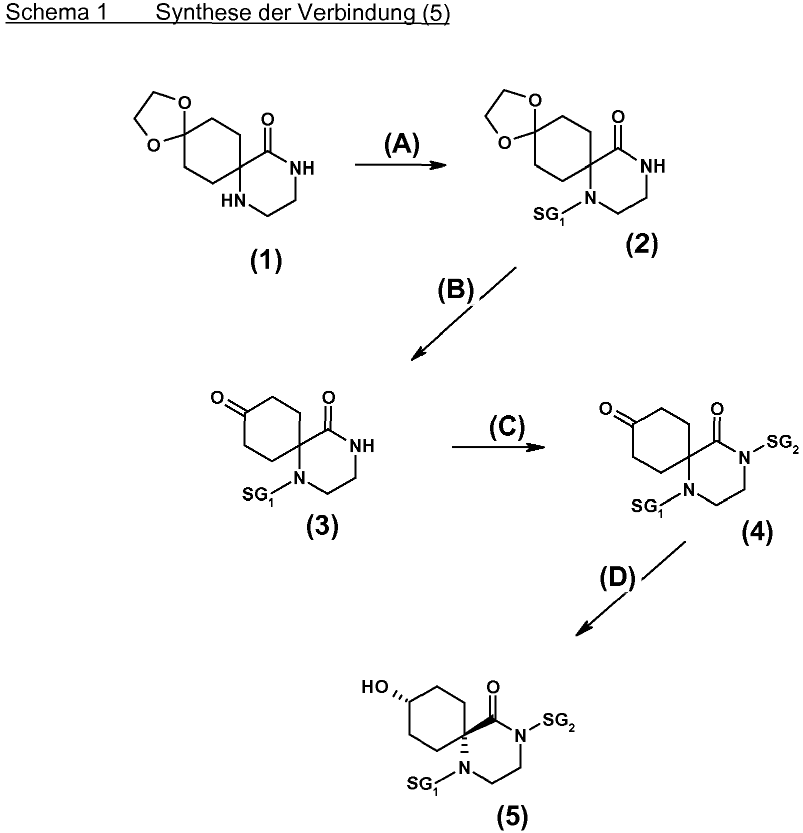

Im Verfahrensschritt **(A)** wird das Edukt (**1**) am Aminstickstoff mit Hilfe von 2,0 bis 3,0 Moläquivalenten Reagenz in Gegenwart von 2,2 bis 3,2 Moläquivalenten Base geschützt. Verfahrensschritt **(B)** beschreibt die wässrig saure Entschützung des Ketals (**2**) zum entsprechenden Keton (**3**) mit Hilfe von 2,0 bis 4,0 Moläquivalenten Säure. Im weiteren Schritt **(C)** wird der noch freie Amidstickstoff mit 1,0 bis 1,5 Moläquivalenten Reagenz in Gegenwart von 2 bis 10 Molprozent Katalysator geschützt. Im letzten Schritt **(D)** erfolgt die katalytische stereoselektive Reduktion der Ketogruppe mit Wasserstoff zu dem *cis-*konfigurierten Alkohol (**5**).

In einer bevorzugten Ausführungsform wird im Verfahrensschritt **(A)** das Edukt (**1**) am Aminstickstoff mit Hilfe von 2,2 Moläquivalenten Trifluoracetanhydrid (TFAA) in Gegenwart von 2,4 Moläquivalenten N-Methylmorpholin (NMM) Trifluoracetyl geschützt. Verfahrensschritt **(B)** beschreibt die wässrig saure Entschützung des Ketals (**2**) zum entsprechenden Keton (**3**) mit Hilfe von 2,8 Moläquivalenten Essigsäure (HOAC). Im weiteren Schritt **(C)** wird der noch freie Amidstickstoff mit 1,2 Moläquibvalenten Di-tert-butyldicarbonat in Gegenwart von 5 Molprozent Dimethylaminopyridin als Carbamat geschützt. Im letzten Schritt **(D)** erfolgt die katalytische stereoselektive Reduktion der Ketogruppe mit Wasserstoff zu dem *cis*-konfigurierten Alkohol (**5**).

Die nachfolgenden Beispiele dienen der Illustration der exemplarisch durchgeführten Verfahren zur Herstellung der Verbindung der Formel (**5**) Diese Beispiele sind als Erläuterung der Erfindung zu verstehen, ohne selbiges auf deren Gegenstand zu beschränken.

### Beispiel 1

### 1,4-Dioxa-9-(2,2,2-trifluoro-acetyl)- 9,12-diaza-dispiro[4.2.5.2]pentadecan-13-on

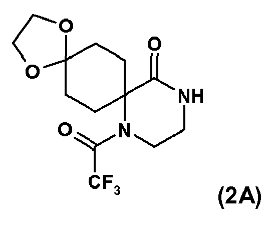

### Verfahrensschritt (A)

Zu 100 g 1,4-Dioxa-9,12-diaza-dispiro[4.2.5.2]pentadecan-13-one in 300 ml 2-Methyltetrahydrofuran werden bei Raumtemperatur (RT entspricht 20 bis 25.°C) 107,2 g N-Methylmorpholin zugetropft. Anschließend wird der Ansatz auf 6 - 10°C abgekühlt und 204,2 g Trifluoracetanhydrid werden zugetropft. Die Temperatur steigt während der Zugabe auf RT an. Nach 120 min werden nacheinander 200 ml Wasser und 450 ml Methylcyclohexan zugegeben. Nach 60 min wird die Suspension auf 0°C abgekühlt. Nach 25 min wird der Niederschlag abfiltriert und mit 100 ml Wasser und 100 ml Methylcyclohexan nacheinander gewaschen. Nach Trocknen bei 50°C im Vakuum erhält man 137,9 g Produkt.
Massenspektrum (ESI⁺): m/z = 323 [M+H]⁺
Smp. 224,8°C

### Beispiel 2

### 1-(2,2,2-trifluoro-acetyl)- 1,4-Diaza-spiro[5.5]undecane-5,9-dion

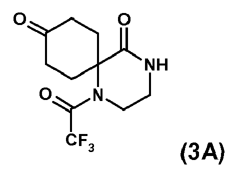

### Verfahrensschritt (B)

Zu 130 g 1,4-Dioxa-9-(2,2,2-trifluoro-acetyl)- 9,12-diaza-dispiro[4.2.5.2]pentadecan-13-on in 390 ml Methanol werden 195 ml Wasser und 65 ml Essigsäure zugefügt. Der Ansatz wird auf 68°C erhitzt und 120 min bei dieser Temperatur gerührt. Anschließend werden 325 ml Lösungsmittel abdestilliert. Die entstandene Suspension wird auf 5°C abgekühlt. Nach 30 min wird der Niederschlag abfiltriert und mit 50 ml tert.-Butylmethylether gewaschen. Nach Trocknen bei 50°C im Vakuum erhält man 101,4 g Produkt.
Massenspektrum (ESI⁺): m/z = 279 [M+H]⁺
Smp. 169,0°C

### Beispiel 3

### 5,9-dioxo-1-(2,2,2-trifluoro-acetyl)-1,4-diaza-spiro[5.5]undecane-4-carboxylic acid tert-butyl ester

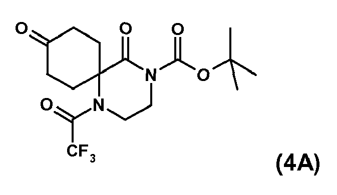

### Verfahrensschritt (C)

Zu einer Mischung aus 90 g 1-(2,2,2-trifluoro-acetyl)-1,4-Diaza-spiro[5.5]undecane-5,9-dion und 1,98 g Dimethylaminopyridin in 180 ml Acetonitril werden bei RT innerhalb von 75 min 84,7 g Di-tert-butyldicarbonat in 90 ml Acetonitril zugetropft. Nach 60 min werden 990 ml Wasser zugegeben und angeimpft. Nach 60 min wird der Niederschlag abfiltriert und mit 180 ml Wasser gewaschen. Nach Trocknen bei 50°C im Vakuum erhält man 113,8 g Produkt.
Massenspektrum (ESI⁺): m/z = 379 [M+H]⁺
Smp. 114,9°C

### Beispiel 4

### (cis)-9-Hydroxy-5-oxo-1-(2,2,2-trifluoro-acetyl)-1,4-diaza-spiro[5.5]undecane-4-carboxylic acid tert-butyl ester

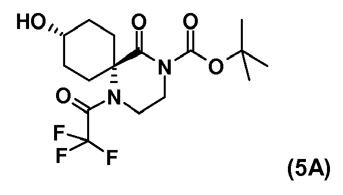

### Verfahrensschritt (D)

Zu 17 g 5,9-dioxo-1-(2,2,2-trifluoro-acetyl)-1,4-diaza-spiro[5.5]undecane-4-carboxylic acid tert-butylester in 170 ml Ethanol werden 5,1 g Raney Nickel zugefügt. Die Reaktionsmischung wird im Autoklav bei 60°C und 50 bar Wasserstoffdruck 10 Stunden hydriert. Anschließend wird der Katalysator abfiltriert und der Filterkuchen mit 40 ml Ethanol nachgewaschen. Es werden 165 ml Ethanol abdestilliert. Zum Rückstand werden 374 ml Toluol zugegeben und weitere 136 ml Lösungsmittel abdestilliert. Die entstandene Lösung wird bei 60°C mit 55 ml Wasser versetzt und die Phasen werden getrennt. Aus der verbleibenden organischen Phase werden weitere 195 ml Lösungsmittel abdestilliert. Die entstandene Suspension wird innerhalb von 120 min auf 2°C abgekühlt. Nach 30 min wird der Niederschlag abfiltriert und zweimal mit je 20 ml Toluol gewaschen. Nach Trocknen bei 50°C im Vakuum erhält man 12,6 g Produkt, das noch ca. 6% trans-Produkt enthält.
Massenspektrum (ESI⁺): m/z = 381 [M+H]⁺
Smp. 163,0°C

### Erstellung von Daten

Zur Erstellung aufgeführten MS Daten wird ein Massenspektrometer *ZQ 2000* (Elektrosprayquelle)/Waters verwendet.

## Patentansprüche

1. Verfahren zur stereoselektiven Herstellung der Verbindung der Formel **(5),** gegebenenfalls in Form ihrer Tautomeren, 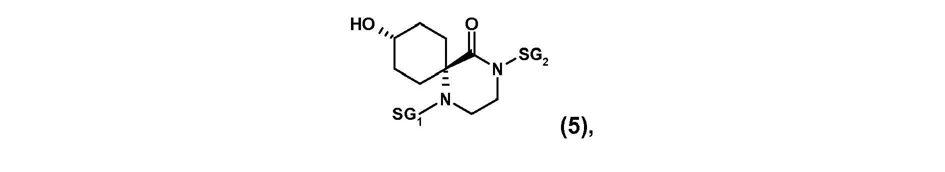 worin
**SG₁** eine Schutzgruppe ausgewählt aus der Gruppe bestehend aus Trifluoracetyl, Acetyl, Benzoyl und Pivaloyl,
**SG₂** eine Schutzgruppe ausgewählt aus der Gruppe bestehend aus tert.-Butoxycarbonyl, Ethoxycarbonyl und Methoxycarbonyl, bedeutet,
**dadurch gekennzeichnet, dass** das Verfahren die Reaktionsschritte **(C)** und **(D)** umfasst, wobei
**(C)** die Umsetzung der Verbindung der Formel **(3)** 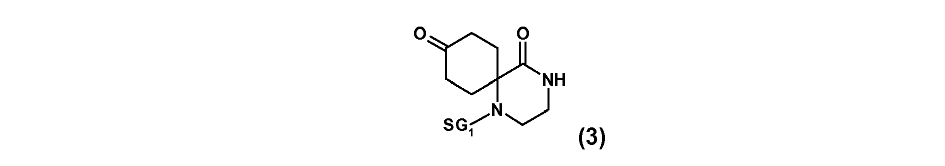 zu einer Verbindung der Formel **(4)** 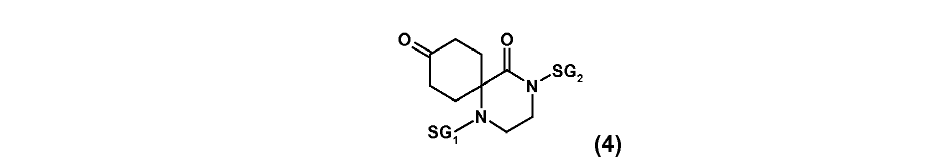 und
**(D)** die Reduktion der Verbindung der Formel **(4)** zu einer Verbindung der Formel **(5)**
bedeutet,
wobei die Verfahrensschritte **(C)** und **(D)** in der genannten Reihenfolge nacheinander erfolgen.

2. Verfahren nach Anspruch 1, **dadurch gekennzeichnet, dass** den Reaktionsschritten **(C)** und **(D)** weitere Reaktionsschritte **(A)** und **(B)** vorausgehen,
wobei
**(A)** die Umsetzung der Verbindung der Formel (**1**) 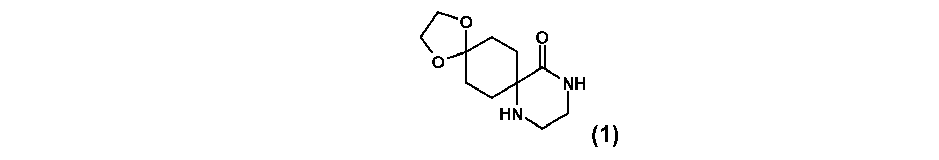 zu einer Verbindung der Formel (2) 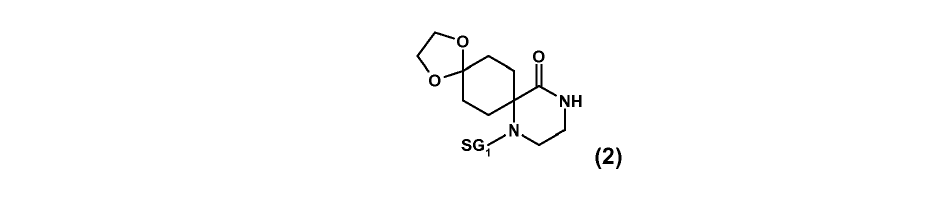 und
**(B)** die Umsetzung der Verbindung der Formel (2) zu einer Verbindung der Formel **(3)** 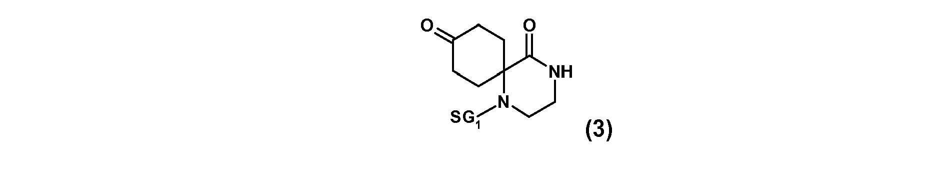 bedeutet,
wobei die Verfahrensschritte **(A), (B), (C)** und **(D)** in der genannten Reihenfolge nacheinander erfolgen.

3. Verfahren nach Anspruch 1 oder 2 zur stereoselektiven Herstellung der Verbindung der Formel **(5A),** gegebenenfalls in Form ihrer Tautomeren, 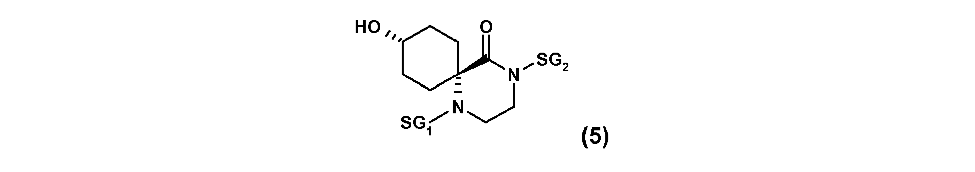 **dadurch gekennzeichnet, dass**
**SG₁** Trifluoracetyl, und
**SG₂** tert.-Butoxycarbonyl,
bedeutet.

4. Intermediat der Formel (**4**) gemäß Anspruch 1 oder 2, gegebenenfalls in Form seiner Tautomeren oder Salze. 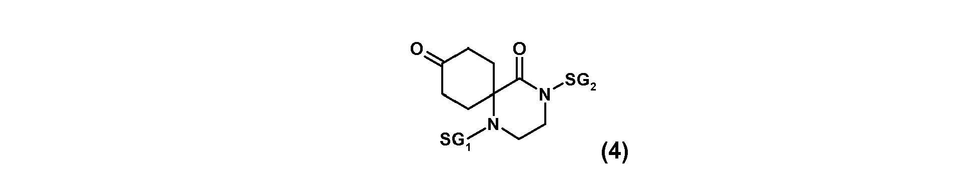 worin
**SG₁** eine Schutzgruppe ausgewählt aus der Gruppe bestehend aus Triflu-oracetyl, Acetyl, Benzoyl und Pivaloyl, und
**SG₂** eine Schutzgruppe ausgewählt aus der Gruppe bestehend aus tert.- Butoxycarbonyl, Ethoxycarbonyl und Methoxycarbonyl, bedeutet,

5. Intermediat der Formel **(4A)** nach Anspruch 4, gegebenenfalls in Form seiner Tautomeren oder Salze.

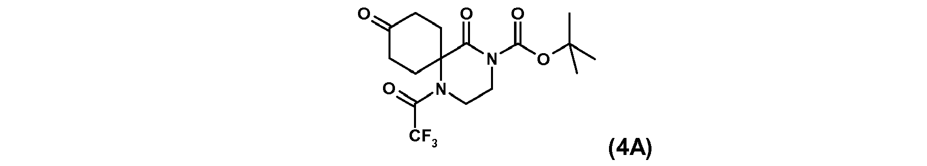

6. Verwendung der Intermediate der Formel **(4)** gemäß Anspruch 4, gegebenenfalls in Form ihrer Tautomeren oder Salze, zur Herstellung einer Verbindung der Formel **(I),** gegebenenfalls in Form ihrer Tautomeren oder Salze,

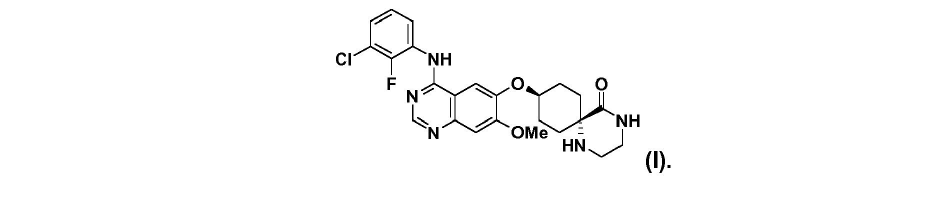

## Claims

1. Method for the stereoselective preparation of the compound of formula **(5),** optionally in the form of the tautomers thereof, 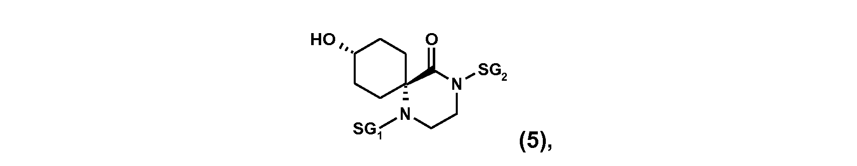 wherein
**SG₁** denotes a protective group selected from among trifluoroacetyl, acetyl, benzoyl and pivaloyl,
**SG₂** denotes a protective group selected from among tert.-butoxycarbonyl, ethoxycarbonyl and methoxycarbonyl,
**characterised in that** the method comprises reaction steps **(C)** and **(D),** where
**(C)** denotes the reaction of the compound of formula **(3)** 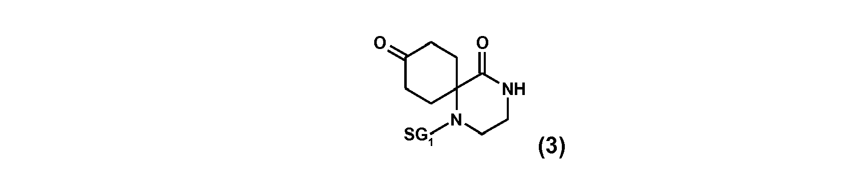 to form a compound of formula **(4)** 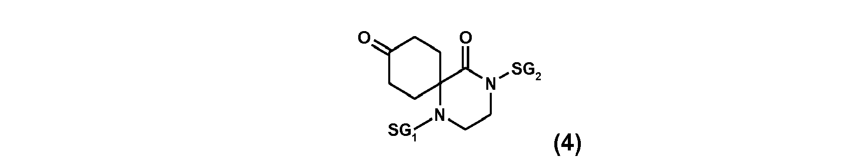 and
**(D)** denotes the reduction of the compound of formula **(4)** to form a compound of formula **(5),**
wherein process steps **(C)** and **(D)** are carried out successively in the order stated.

2. Method according to claim 1, **characterised in that** reaction steps **(C)** and **(D)** are preceded by further reaction steps **(A)** and **(B),**
wherein
**(A)** denotes the reaction of the compound of formula **(1)** 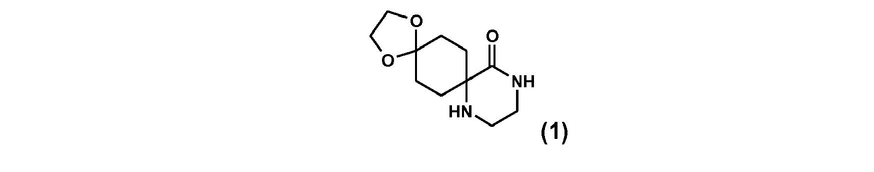 to form a compound of formula **(2)** 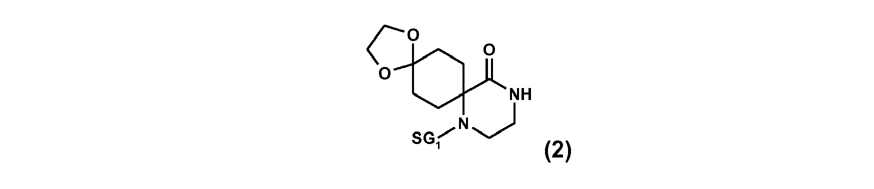 and
**(B)** denotes the reaction of the compound of formula **(2)** to form a compound of formula **(3)** 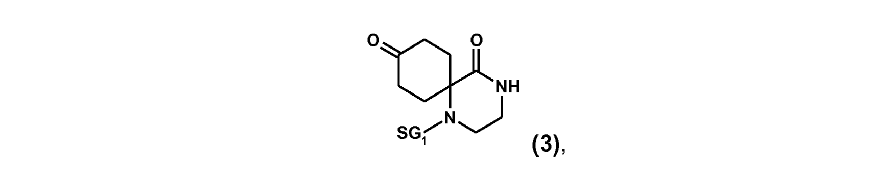 wherein process steps **(A), (B), (C)** and **(D)** are carried out successively in the order stated.

3. Method according to claim 1 or 2 for the stereoselective preparation of the compound of formula (**5A**), optionally in the form of the tautomers thereof, 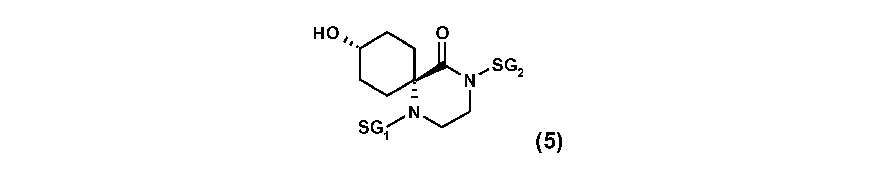 **characterised in that**
**SG₁** denotes trifluoroacetyl, and
**SG₂** denotes tert.-butoxycarbonyl.

4. Intermediate of formula **(4)** according to claim 1 or 2, optionally in the form of the tautomers or salts thereof, 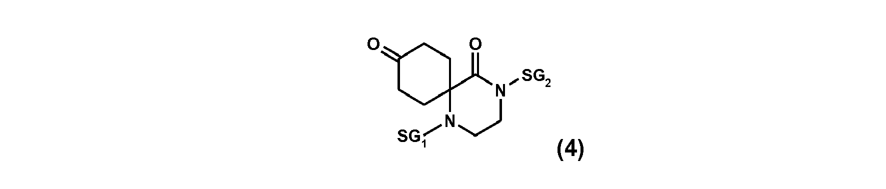 wherein
**SG₁** denotes a protective group selected from among trifluoroacetyl, acetyl, benzoyl and pivaloyl, and
**SG₂** denotes a protective group selected from among tert.- butoxycarbonyl, ethoxycarbonyl and methoxycarbonyl.

5. Intermediate of formula **(4A)** according to claim 4, optionally in the form of the tautomers or salts thereof.

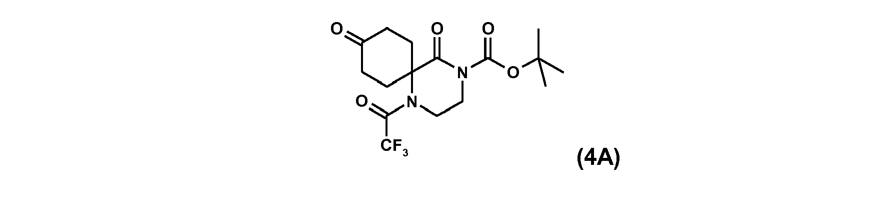

6. Use of the intermediates of formula **(4)** according to claim 4, optionally in the form of the tautomers or salts thereof, for preparing a compound of formula **(I),** optionally in the form of the tautomers or salts thereof,

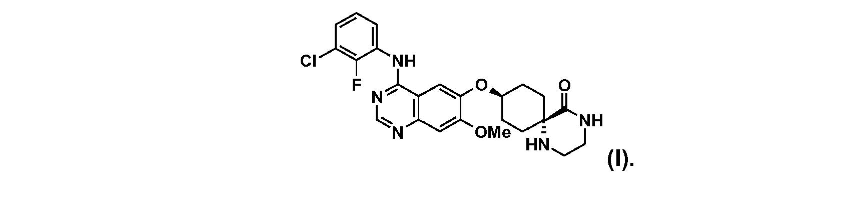

## Revendications

1. Procédé de préparation stéréosélective du composé de formule (5), éventuellement sous la forme de ses tautomères, 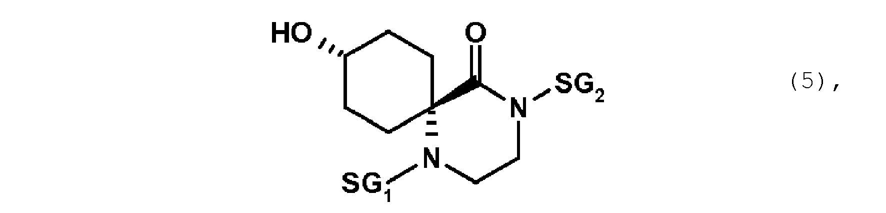 dans laquelle
SG₁ représente un groupe protecteur choisi dans le groupe consistant en un trifluoroacétyle, un acétyle, un benzoyle et un pivaloyle,
SG₂ représente un groupe protecteur choisi dans le groupe consistant en un tert-butoxycarbonyle, un éthoxycarbonyle et un méthoxycarbonyle,
**caractérisé en ce que** le procédé comprend les étapes de réaction (C) et (D),
(C) représentant la conversion du composé de formule (3) 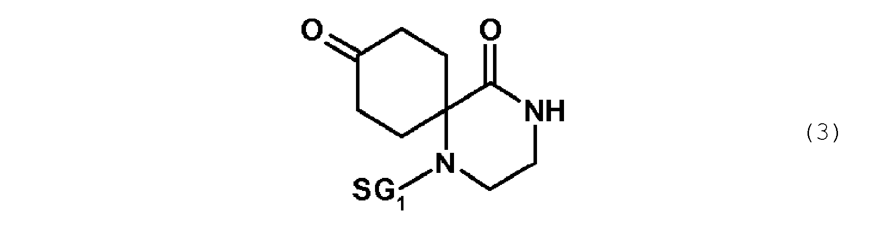 en un composé de formule (4) 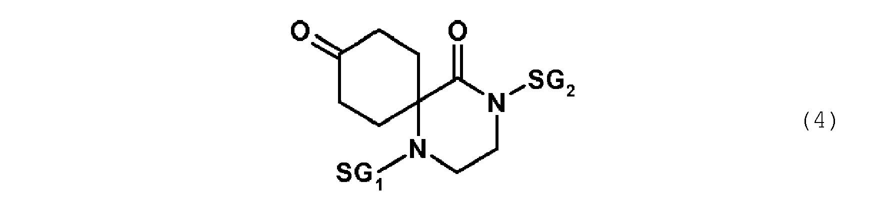 et
(D) représentant la réduction du composé de formule (4) en un composé de formule (5),
les étapes de procédé (C) et (D) s'effectuant consécutivement dans cet ordre.

2. Procédé selon la revendication 1, **caractérisé en ce que** les étapes de réaction (C) et (D) sont précédées par d'autres étapes de réaction (A) et (B),
(A) représentant la conversion du composé de formule (1) 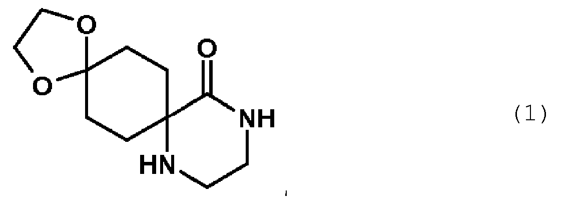 en un composé de formule (2)
et 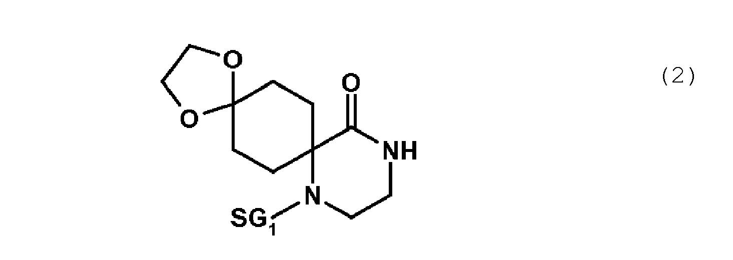
(B) représentant la conversion du composé de formule (2) en un composé de formule (3) 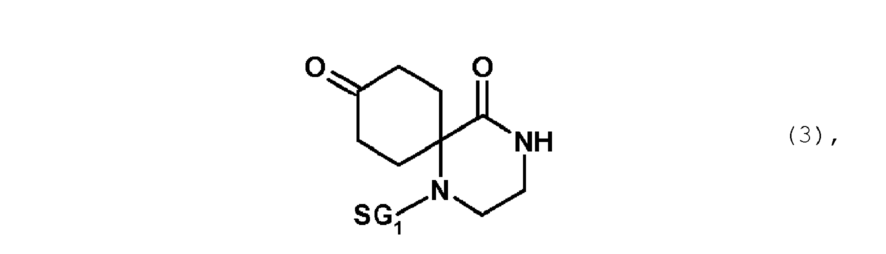 les étapes de procédé (A), (B), (C) et (D) s'effectuant consécutivement dans cet ordre.

3. Procédé selon la revendication 1 ou 2 de préparation stéréosélective du composé de formule (5A), éventuellement sous la forme de ses tautomères, 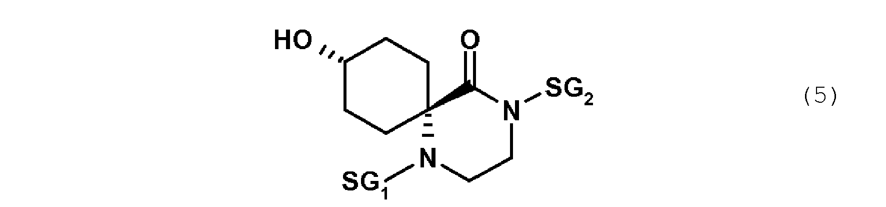 **caractérisé en ce que**
SG₁ représente un trifluoracétyle, et
SG₂ représente un tert-butoxycarbonyle.

4. Intermédiaire de formule (4) selon la revendication 1 ou 2, éventuellement sous la forme de ses tautomères ou de ses sels, 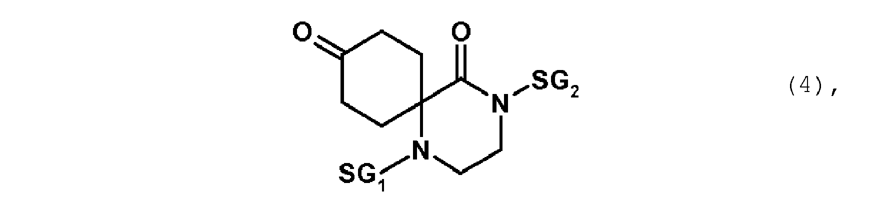 dans laquelle
SG₁ représente un groupe protecteur choisi dans le groupe consistant en un trifluoroacétyle, un acétyle, un benzoyle et un pivaloyle, et
SG₂ représente un groupe protecteur choisi dans le groupe consistant en un tert-butoxycarbonyle, un éthoxycarbonyle et un méthoxycarbonyle.

5. Intermédiaire de formule (4A) selon la revendication 4, éventuellement sous la forme de ses tautomères ou de ses sels.

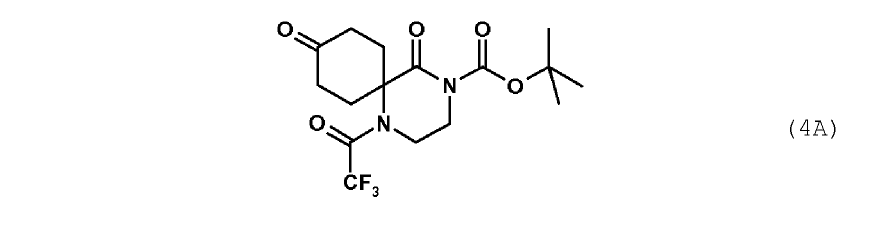

6. Utilisation de l'intermédiaire de formule (4) selon la revendication 4, éventuellement sous la forme de ses tautomères ou de ses sels, pour la préparation d'un composé de formule (I), éventuellement sous la forme de ses tautomères ou de ses sels,
